**Europäisches Patentamt**

**European Patent Office**

**Office européen des brevets**

(11) Veröffentlichungsnummer: **0 332 871**
**A2**

## (12) EUROPÄISCHE PATENTANMELDUNG

(21) Anmeldenummer: **89102672.6**

(22) Anmeldetag: **16.02.89**

(51) Int. Cl.⁴ **A61B 17/22**

(30) Priorität: **16.03.88 DE 3808783**

(43) Veröffentlichungstag der Anmeldung:
**20.09.89 Patentblatt 89/38**

(84) Benannte Vertragsstaaten:
**CH ES FR GB IT LI**

(71) Anmelder: **DORNIER MEDIZINTECHNIK GMBH**
**Postfach 1128**
**D-8034 Germering 1(DE)**

(72) Erfinder: **Ganter, Doris**
**Steinbergstrasse 37**
**D-8034 Germering(DE)**
Erfinder: **Hermeking, Hajo, Dr.rer.nat.**
**Spitzweg 16**
**D-8034 Germering(DE)**
Erfinder: **Zeitler, Rudolf, Dr.**
**Julius-Staerlin-Strasse 1**
**D-8035 Gauting(DE)**
Erfinder: **DiMonda, Richard**
**824 Livingston Court**
**Marietta, GA 30067(US)**
Erfinder: **Brendel, Walter, Prof.Dr.**
**Egenhofenstrasse 17a**
**D-8033 Planegg(DE)**
Erfinder: **Delius, Michael, Dr.**
**Schraudolphstrasse 12**
**D-8000 München 40(DE)**

(74) Vertreter: **Landsmann, Ralf, Dipl.-Ing.**
**c/o DORNIER GMBH Postfach 1420**
**D-7990 Friedrichshafen 1(DE)**

(54) **Steinzerkleinerung durch kombinierte Behandlung.**

(57) Verfahren zum Zerkleinern von Konkrementen in Körpern von Lebewesen, gekennzeichnet durch die Kombination extrakorporal erzeugter Stosswellen und Ultraschallbehandlung. Bevorzugt wird die Behandlung durch die orale Gabe von steinlösenden Medikamenten unterstützt.

Fig.3

## Steinzerkleinerung durch kombinierte Behandlung

Die Erfindung betrifft ein Verfahren zur Zerkleinerung von Konkrementen in Körpern von Lebewesen, eine Vorrichtung zur Durchführung des Verfahrens und die gewerbliche Verwendung von Erzeugnissen zu den genannten Verfahren.

Aus der EP 225 104 A1 ist es bekannt, zur Gallensteinzerkleinerung Ultraschallfelder zusammen mit einem Lysemittel zu verwenden, das mittels eines Katheders eingeführt wurde.

Es wurde bereits vorgeschlagen (deutsche Patentanmeldung P 37 08 325), Gallensteine mit Stosswellen zu zerkleinern und anschliessend mit Stosswellen niedriger Energie die Bruckstücke in einem Lysemittel zu bewegen, welches mit einem Katheder eingegeben wurde. Anstelle der Stosswellen wird auch vorgesehen, Ultraschallwellen geringerer Stärke zu verwenden.

Aufgabe der Erfindung ist es, ein Verfahren vorzuschlagen, mit dem Konkremente in Körpern von Lebewesen, insbesondere Nierensteine, Gallensteine oder Gallenblasensteine, schneller und für die Patienten möglichst wenig belastend, zerkleinert werden können.

Diese Aufgabe wird erfindungsgemäss gelöst von Verfahren mit den in den Ansprüchen angegebenen Schritten. Gewerbliche Verwendungen und Vorrichtungen sind Gegenstände von Unteransprüchen.

Kern der Erfindung ist die Kombination von zwei an sich schon bekannten Behandlungsmethoden, nämlich der extrakorporalen Stosswellen-Lithotripsie und der Ultraschallbehandlung gleichzeitig oder hintereinander. Es wurde gefunden, dass die Kombination von Stosswellen- und Ultraschallbehandlung zu einer wesentlich schnelleren Auflösung der Steine führt, als dies für beide einzelne Maßnahmen zu erwarten war. Der synergistische Effekt beruht vermutlich darauf, dass die Stosswellen besonders effektiv arbeiten, wenn sich in der Steinumgebung Kavitationskeime befinden, die z.B. durch Ultraschall oder durch chemische Mittel, die die Kavitationsbildung verbessern, erzeugt wurden.

Bei der Erfindung handelt es sich vorzugsweise um ein nichtinvasives Verfahren zur Behandlung von Steinleiden, insbesondere von Gallensteinen, Gallenblasensteinen oder Nierensteinen. Ein Vorteil liegt darin, dass durch die Kombination von Stosswellen und Ultraschallbehandlung - bevorzugt in Verbindung mit einer Litholyse - die Steine rasch zerkleinert werden und der Zeitraum bis zum Erreichen völliger Steinfreiheit erheblich verkürzt wird. Die Erfindung zeichnet sich gegenüber den separaten Verfahren dadurch aus, dass durch geeignete kombination der Methoden eine optimale Wirkung erzielt wird. Daraus resultiert eine erheblich reduzierte Belastung des Patienten und eine Erweiterung des Indikationsbereiches.

Bevorzugt wird vor, während und nach der Behandlung der lithogene Index der das Konkrement umgebenden Flüssigkeit durch orale Medikamentengabe erniedrigt; das heisst es wird z.B. in der Gallenblase eine cholesterinuntersättigte Gallenflüssigkeit erzeugt. Dazu finden derzeit Chenodesoxycholsäure, Ursodesoxycholsäure und eine Kombination der beiden Mittel Verwendung.
Möglich ist jedoch auch, die Umgebung der Steine durch eine entsprechende Diät oder - invasiv - durch Einspritzen von Wasser in die Gallenblase zu verändern. Das heisst, bereits durch eine diätetische Behandlung kann die Umgebung des Steins dahingehend verändert werden, dass die Auflösung des Steins durch Ultraschall wesentlich schneller erfolgt.

Die solitären oder multiplen Steine werden durch die extrakorporal erzeugten Stosswellen zerkleinert. Die Zertrümmerungswirkung und die Auflösungsgeschwindigkeit der verbleibenden Steinfragmente wird durch Anwendung von Ultraschall hoher Intensität erhöht. Hierbei kann auf verschiedene Weise vorgegangen werden:
- Stosswellen-Applikation unter ständiger Ultraschallexposition des Steines
- Stosswellen-Applikation mit Ultraschallexposition in den Behandlungspausen
- Stosswellen-Applikation mit anschliessender Ultraschallexposition.

Die Ultraschallbehandlung im Anschluß an die Stosswellenbehandlung kann sich über mehrere Tage erstrecken und mehrmals täglich wiederholt werden.

Bei dem Ultraschallschwinger kann es sich sowohl um einen fokussierenden, teilfokussierenden oder planaren Schwinger handeln. Auf die Steinumgebung fokussierte Stosswellen haben besonders geringe Nebenwirkungen. Die Ultraschallexposition kann entweder kontinuierlich erfolgen oder intermittierend, das heisst, die Pulslänge, die Periodendauer sowie die Wiederholfrequenz sind frei wählbar, wodurch sich eine optimale Anpassung an die Steine erzielen lässt.

Die Intensität des Schwingers liegt im therapeutischen Bereich, sollte aus Gründen der Gewebeschonung einen Spitzenwert von ca. 15 W/cm$^2$ aber nicht überschreiten. Die Arbeitsfrequenz liegt im Bereich zwischen 10 Hz und 500 kHz, bevorzugt zwischen 10 kHz und 500 kHz. Niederfrequenter Ultraschall eignet sich be-sonders in Verbindung mit chemischen Lysemitteln.

Bei dem Ultraschallschwinger kann es sich um einen von der Stosswellenquelle getrennten Schwinger handeln, oder um einen Schwinger, mit dem wahlweise Stosswellen hoher Energie, Stosswellen niedriger Energie oder sinusförmige Ultraschallwellen generiert werden können.

Neben sinusförmigen Ultraschallwellen können auch Stosswellen niederer Energie und hoher Wiederholfrequenz (60 Hz bis 200 kHz) appliziert werden.

Es wurde gefunden, dass die Fragmentation von Gallenblasensteinen und von Nierensteinen durch Stosswellen stark von dem Umgebungsdruck abhängig ist. Diese Abhängigkeit lässt sich damit erklären, dass die Zertrümmerung der genannten Steine wesentlich durch Kavitationsbildung und anschliessenden Kavitationsblasenkollaps im Stein und seiner unmittelbaren Umgebung abhängt. Die Kavitationswirkung ist damit wesentlich von der Kavitationskeimdichte im Stein und seiner Umgebung unmittelbar während der Stosswellenanwendung abhängig.

Erfindungsgemäss wird die Kavitationskeimdichte des den Stein umgebenden Mediums durch geeignete Wahl der quasistationären lokalen Zustandsparameter optimiert, insbesondere durch die geeignete Wahl des umgebenden Mediums. Die relevanten Parameter sind:
- niedriger Dampfdruck, geringe Viskosität,
- Impfung mit Kavitationskeimen,
- Gabe von Kontrastmitteln mit Neigung von Kavitationsbildung,
- Ultraschallbeschallung.

Auch die Kavitationskeimdichte im Stein wird durch vorherige Beschallung mit Ultraschall erhöht. Die Kavitationskeimdichte wird auch wesentlich durch die Stosswellenapplikation während der Ultraschallbeschallung beeinflusst.

Eine andere Erklärung für die überraschend gute Wirkung der erfindungsgemässen Kombination könnte sein, dass Ultraschall und Stosswelle lokal im Stein durch konstruktive oder destruktive Interferenz zu erhöhtem Druck und zu höherem Zug- bzw. Druckgradienten führen. Die Stosswellen und die Ultraschallwellen können aus der gleichen oder aus verschiedenen Richtungen auf den Stein geleitet werden. Durch Variation der Arbeitsfrequenz des Ultraschallschwingers kann durch Erzeugung von Resonanzeffekten in den Kavitationsblasen bzw. im Stein eine Verbesserung der Steinauflösung erzielt werden.

Neben Stosswellen hoher Energie, die im Stein Drucke in k-bar-Bereich erzeugen, können auch Stosswellen niedriger Energie, die Drucke im Bereich mehrerer bar erzeugen, verwendet werden. Allerdings sollten die schwächeren Stosswellen dann mit höheren Wiederholfrequenzen verwendet werden.

Die Erfindung wird anhand dreier Figuren näher erläutert.

Es zeigen:

Figur 1 einen fokussierenden, piezoelektrischen Schwinger,

Figur 2 die vom Piezoschwinger erzeugten Signalformen und

Figur 3 die kombinierte Anwendung von Stosswellen und Ultraschall.

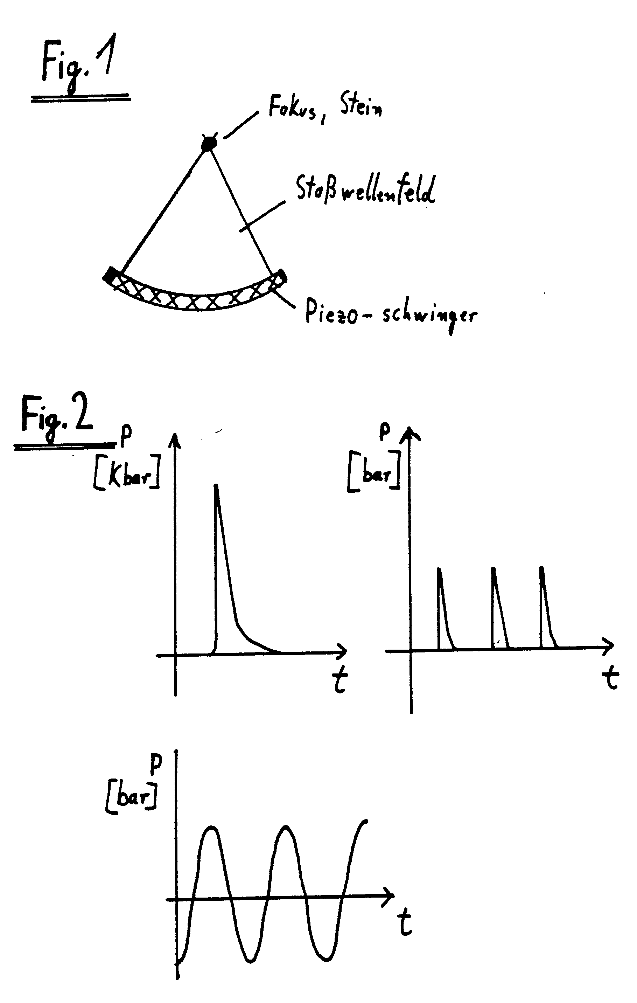

Figur 1 zeigt schematisch einen piezoelektrischen Schwinger, der ein fokussiertes Schallfeld auf den Stein leitet, der sich im Fokus des Schwinger befindet.

Figur 2 zeigt drei verschiedene Signalformen, die von dem in Figur 1 gezeigten Schwinger erzeugt werden können. Links oben ist der Druckverlauf einer Stosswelle gezeigt, die Maximaldrucke im Fokus liegen dabei im Bereich von kbar. Rechts oben ist der Druckverlauf von mehreren Stosswellen niedrigerer Energie gezeigt. Die Drucke im Fokus liegen im Bereich von mehreren oder von zigbar, die Wiederholfrequenz ist dafür wesentlich höher als bei der einzelnen, starken Stosswelle, wie sie links gezeigt ist.

Links unten ist der Druckverlauf einer Ultraschallwelle gezeigt. Die Drucke bewegen sich im bar-Bereich.

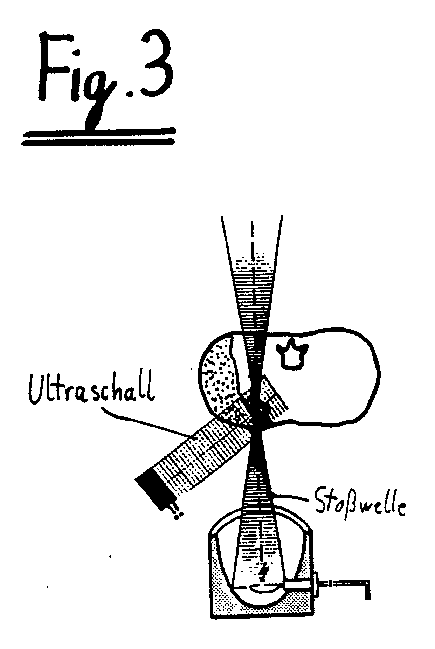

Figur 3 zeigt unten eine Stosswellenquelle, die Stosswellen werden hier durch Funkenentladung erzeugt und über den Reflektor auf den Stein im Körper des Patienten geleitet. Links ist ein Ultraschallsender gezeigt, der hier ein unfokussiertes Ultraschallfeld in den Steinbereich sendet. Beide Geräte können unabhängig voneinander oder miteinander korreliert betrieben werden. Die Wahl der Eintrittsfenster in den Körper des Patienten ist beliebig. Die Einleitung der Stosswellen oder der Ultraschallwellen erfolgt wie üblich über Kissen oder mittels eines Flüssigkeitsbades.

**Ansprüche**

1. Verfahren zum Zerkleinern von Konkrementen in Körpern von Lebewesen, **gekennzeichnet** durch die Kombination:
- extrakorporal erzeugte Stosswellen und
- Ultraschallbehandlung.

2. Verfahren nach Anspruch 1, dadurch gekennzeichnet, dass zusätzlich ein steinauflösendes chemisches Mittel, insbesondere oral, gegeben wird.

3. Verfahren nach Anspruch 1 oder Anspruch 2, dadurch gekennzeichnet, dass das Konkrement gleichzeitig mit Stosswellen und Ultraschall behandelt wird.

4. Verfahren nach Anspruch 1 oder Anspruch 2, dadurch gekennzeichnet, dass das Konkrement zeitlich versetzt mit Stosswellen und mit Ultraschall behandelt wird.

5. Verfahren nach Anspruch 4, dadurch gekennzeichnet, dass di Ultraschallbehandlung nach der Stosswellenbehandlung erfolgt.

6. Verfahren nach einem der Ansprüche 2 bis 5, dadurch gekennzeichnet, dass die Gabe des Lysemitteis oral erfolgt, wobei bei der Zerkleinerung Gallensteinen Mittel gegeben werden, die eine Cholesterin untersättigte Gallenflüssigkeit erzeugen.

7. Verfahren nach einem der Ansprüche 2 bis 5, dadurch gekennzeichnet, dass das Lysemittel oral gegeben wird, wobei ein solches Mittel verwendet wird, das eine Kavitationsbildung im Konkrement oder in der Konkrementumgebung verursachen oder verbessern kann.

8. Gewerbliche Verwendung einer extrakorporalen Stosswellenquelle, einer Ultraschall-Behandlungsvorrichtung und von steinauflösenden Medikamenten, insbesondere oral, zur Zerkleinerung von Konkrementen in Körpern von Lebewesen.

9. Verwendung von steinauflösenden Mitteln zur Herstellung von Medikamenten zur extrakorporalen Steinzerkleinerung mit Stosswellen und Ultraschall.

10. Verwendung einer extrakorporalen Stosswellenquelle und eines Ultraschallgeräts zur Herstellung eines Lithotripters, der gleichzeitig oder zeitlich versetzt Stosswellen und Ultraschall in den Körper eines Lebewesens einleitet.

11. Vorrichtung zum Zerkleinern von Konkrementen in Körpern von Lebewesen, dadurch gekennzeichnet, dass eine extrakorporale Stosswellenquelle und ein Ultraschall-Behandlungsgerät vorgesehen sind, die gleichzeitig oder zeitlich versetzt arbeiten.

# Fig. 1

Fokus, Stein

Stoßwellenfeld

Piezo-schwinger

# Fig. 2

# Fig.3

Ultraschall

Stoßwelle